**Europäisches Patentamt**

**European Patent Office**

**Office européen des brevets**

(19)

(11) Veröffentlichungsnummer: **0 274 701 B1**

## (12) EUROPÄISCHE PATENTSCHRIFT

(45) Veröffentlichungstag der Patentschrift: **04.12.91**

(51) Int. Cl.⁵: **A61K 7/13**

(21) Anmeldenummer: **87118746.4**

(22) Anmeldetag: **17.12.87**

(54) **Haarfärbemittel und Verfahren zur Haarfärbung.**

(30) Priorität: **16.01.87 DE 3701098**

(43) Veröffentlichungstag der Anmeldung:
**20.07.88 Patentblatt 88/29**

(45) Bekanntmachung des Hinweises auf die
Patenterteilung:
**04.12.91 Patentblatt 91/49**

(84) Benannte Vertragsstaaten:
**AT BE CH DE ES FR GB GR IT LI NL SE**

(56) Entgegenhaltungen:
**DE-A- 1 229 980**
**DE-A- 1 242 794**
**US-A- 2 817 342**
**US-A- 3 839 220**

(73) Patentinhaber: **HANS SCHWARZKOPF GmbH**
**Hohenzollernring 127/129**
**W-2000 Hamburg 50(DE)**

(72) Erfinder: **Hoppe, Udo, Dr.**
**Lottbeker Weg 7**
**W-2000 Hamburg 65(DE)**
Erfinder: **Körbächer, Klaus, Dr.**
**Friedrich Ebert-Allee 126**
**W-2000 Schenefeld(DE)**
Erfinder: **Schrader, Karl-Heinz**
**Max Planck-Str. 6**
**W-3540 Holzminden(DE)**

EP 0 274 701 B1

**Beschreibung**

Die Erfindung betrifft ein Haarfärbemittel auf der Basis von Oxydationsfarben, bei dem der für die Färbung erforderliche alkalische pH-Wert erst während der Anwendung auf enzymatischem Wege durch Einwirkung von Urease auf Harnstoff erzeugt wird und ein Verfahren zum Färben von Haaren mit Hilfe eines derartigen Haarfärbemittels.

In erheblichem Umfang werden zum Färben von Haaren die sogenannten Oxydationsfarben eingesetzt, da diese im Färbeergebnis intensive Farben von hoher Farbechtheit liefern. Dabei entstehen die Oxydationsfarben während des Färbevorgangs durch oxydative Kupplung einer Entwicklerkomponente und einer Kupplerkomponente. Derartige Kuppler- und Entwicklerkomponenten zur Erzielung verschiedenster Farbtöne, im folgenden zusammenfassend als Haarfarbzwischenprodukte bezeichnet, sind zahlreich in der Literatur beschrieben.

Obwohl die oxydative Kupplung, d.h. die Entwicklung der Färbung, im Prinzip auch durch Luftsauerstoff erfolgen kann, werden in den meisten Fällen chemische Oxydationsmittel eingesetzt. Bewährt haben sich dabei insbesondere Oxydationsmittel auf der Basis von Wasserstoffperoxyd, neben Wasserstoffperoxyd selbst vorzugsweise dessen Additionsverbindungen mit Harnstoff, Melamin oder Natriumborat.

In US-A-3 839 220 liegen die Reaktionspartner für 2B. Haarfärbemittel auf der Basis von Oxydationsfarben unter Druck mikroverkapselt vor. Bei Freisetzung auf Atmosphären druck besten die Mikrokapseln sodaß die Reaktionspartner zur Reaktion gelangen.

Für die praktische Anwendung als Haarfärbemittel werden die Farbstoffzwischenprodukte in eine geeignete kosmetische Grundlage eingearbeitet, die je nach Art des gewünschten Endproduktes eine Lösung, eine Creme oder ein Gel sein kann. Diese Zubereitung wird dann unmittelbar vor der Anwendung auf dem Haar mit dem Oxydationsmittel versetzt.

Derartige Haarfärbemittel gemäß dem Stand der Technik sind normalerweise auf einen alkalischen pH-Wert eingestellt, vorzugsweise durch einen Zusatz von Ammoniak oder organischen Aminen wie z.B. Monoethanolamin.

Dieser alkalische pH-Wert üblicher Haarpflegemittel führt zwar zu einem leichten Ablauf der farbbildenden Oxydationsreaktion und aufgrund der Quellung des Haares im alkalischen Medium auch zu einer guten Aufnahme der Haarfarbe. Er weist jedoch auch nicht unerhebliche Nachteile auf: Durch die erhebliche Abweichung vom schwach sauren, hautphysiologischen pH-Wert wird die Kopfhaut und das Haar stark beansprucht. Aus dem gleichen Grund ist auch ein Auftragen eines derartigen Haarfärbemittels mit den ungeschützten Händen nicht ohne weiteres möglich, was die Anwendung auf dem eigenen Haar und außerhalb eines Frisiersalons erschwert.

Der vorliegenden Erfindung lag daher die Aufgabe zugrunde, ein Haarfärbemittel auf der Basis von Oxydationsfarben zu schaffen, welches gegenüber dem Stand der Technik bei mindestens gleicher oder besserer Qualität des Färbeergebnisses eine geringere Alkalibelastung des Haares und damit auch eine größere Milde und bessere Verträglichkeit auf der Haut aufweist.

Es wurde nun überraschend gefunden, daß sich ein derartiges Haarfärbemittel schaffen läßt, indem man eine Zusammensetzung wählt, bei der, ausgehend von einer im sauren Bereich gepufferten Grundmasse, der alkalische pH-Wert erst während des Färbevorgangs auf enzymatischem Wege durch die Einwirkung von Urease auf Harnstoff erzeugt wird.

Gegenstand der Erfindung ist daher ein Haarfärbemittel auf der Basis von Oxydationsfarben, gekennzeichnet durch

a) eine mittels eines geeigneten Puffergemisches im sauren Bereich gepufferte Grundmasse, die außerdem die Haarfarbzwischenprodukte und Harnstoff enthält,
b) Urease,
c) ein in einem gegen säurebeständigen, in alkalischem Medium löslichen Material mikroverkapseltes, Wasserstoffperoxid abgebendes Oxydationsmittel,
wobei die drei Komponenten (a), (b) und (c) erst unmittelbar vor dem Färbevorgang gemischt werden.

Bei der Grundmasse des erfindungsgemäßen Haarfärbemittels handelt es sich je nach Art des gewünschten Endproduktes beispielsweise um ein Gel, eine Creme oder eine tensidhaltige, schäumende Lösung, wie z.B. ein Shampoo. Diese Grundmasse enthält die aus dem Stand der Technik bekannten und für die Anwendung auf dem Haar geeigneten Bestandteile in für diese Zwecke üblichen Mengen. Bestandteile derartiger Grundmassen sind z.B.:

- Netz- und Emulgiermittel wie ionische und nicht ionische Tenside, beispielsweise Alkylsulfonate, Fettalkoholsulfate, Fettalkoholpolyglykolethersulfate, Ethoxylierungsprodukte von Fettalkoholen, Fettsäuren oder Alkylphenolen, Sorbitanfettsäureester, Fettsäurepartialglyceride und Fettsäurealkanolamide.

2

- Verdickungsmittel, beispielsweise Cellulosederivate, Stärke, Fettalkohole, Paraffinöle und Fettsäuren.
- Reduktionsmittel, z.B. Natriumsulfit
- Parfümöle
- haarpflegende Zusätze, z.B. wasserlösliche kationische Polymere, Proteinderivate, Lecitin, Cholesterin und Pantothensäure
- Lösungsmittel, beispielsweise Wasser oder niedere Alkohole.

Erfindungsgemäß wird die Grundmasse mit Hilfe eines Puffergemisches auf einen sauren pH-Wert, vorzugsweise im pH-Wert-Bereich von 4,9 - 6,9, insbesondere im pH-Wert-Bereich 6,0 - 6,8 eingestellt. Der Anteil des Puffergemisches an der Grundmasse beträgt vorzugsweise 2 - 25 Gew.-%. Vorzugsweise geeignete Puffergemische sind Citrat- oder Phosphatpuffer, wobei die Bestandteile des Puffergemisches den übrigen Bestandteilen der Grundmasse entweder als reine Stoffe zugemischt oder als wässrige Lösung zugesetzt werden können.

Insbesondere wird als Puffergemisch eine wäßrige Lösung eingesetzt, die 0,7 - 1,0 Gew.-% Citronensäure und 3,4 - 4,3 Gew.-% Diammoniumhydrogenphosphat enthält, in einer besonders bevorzugten Ausführungsform mit einem Verhältnis Citronensäure zu Diammoniumhydrogenphosphat von 1 : 4,4 - 1 : 4,9.

Zusätzlich enthält die Grundmasse des erfindungsgemäßen Haarfärbemittels Harnstoff in einer Menge von vorzugsweise 2 - 20 Gew.-%, insbesondere 2 bis 6 Gew.-%.

Weiterhin enthält die Grundmasse die Haarfarbzwischenprodukte, und zwar erfindungsgemäß vorzugsweise 0,01 bis 5,0 Gew.-%, insbesondere 0,05 bis 3,0 Gew.-% einer oder mehrerer üblicher Entwicklerkomponenten und vorzugsweise 0,01 bis 3,0 Gew.-%, insbesondere 0,05 bis 2,0 Gew.-% einer oder mehrerer üblicher Kupplerkomponenten. Dabei beträgt das Mol-Verhältnis von Entwicklerkomponente bzw. -komponenten zu Kupplerkomponente bzw. -komponenten im erfindungsgemäßen Haarfärbemittel in der Regel etwa 1:1, ein Überschuß des einen oder anderen Haarfarbzwischenprodukts ist jedoch nicht nachteilig.

Im Rahmen der Erfindung sind prinzipiell alle üblichen, dem Fachmann bekannten Entwickler- und Kupplerkomponenten einsetzbar, bevorzugt werden jedoch je nach gewünschter Farbgebung die Entwicklerkomponente(n) ausgewählt aus der Gruppe der p-Toluylendiamine, der p-Phenylendiamine, der 2,5-Diaminopyridine oder der p-Aminophenole als freie Base oder in Form eines kosmetisch akzeptablen Salzes und die Kupplerkomponente(n) aus der Gruppe der Resorcine, der m-Aminophenole, der m-Phenylendiamine, der 2,3-Diamino-6-methoxipyridine oder alpha-Naphtol.

Zur Erzielung spezieller Farbnuancen kann es außerdem zweckmäßig sein, der erfindungsgemäßen Grundmasse zusätzlich zu den bereits genannten Haarfarbzwischenprodukten geringe Mengen eines oder mehrerer direktziehender Haarfarbstoffe zuzusetzen.

Die zweite Komponente des erfindungsgemäßen Haarfärbemittels ist das Enzym Urease, in einer Menge von vorzugsweise 0,01 bis 0,20 Gewichtsteilen, insbesondere 0,05 bis 0,15 Gewichtsteilen pro 100 Gewichtsteile der Grundmasse. In einer speziellen Ausführungsform der Erfindung, die sich besonders für Färbungen beim Vorliegen von "fetten" Haarschäften eignet, wird als Komponente (b) das Enzym Urease, vorzugsweise in den vorstehend angegebenen Mengen, im Gemisch mit dem Enzym Lipase eingesetzt, wobei die Lipase in einer Menge von vorzugsweise 0,01 bis 0,1 Gewichtsteilen, insbesondere 0,03 bis 0,08 Gewichtsteilen, pro 100 Gewichtsteile Grundmasse eingesetzt wird, wobei die beiden Enzyme vorzugsweise trocken vermischt werden. In einer weiteren speziellen Ausführungsform der Erfindung kann das Enzym bzw. das Enzym-Gemisch mit einem inerten Feststoff, z.B. feinverteiltem Siliciumdioxyd, verdünnt eingesetzt werden.

Die dritte Komponente des erfindungsgemäßen Haarfärbemittels besteht aus einem mikroverkapselten, Wasserstoffperoxid abgebenden Oxidationsmittel, vorzugsweise einem Anlagerungsprodukt von Wasserstoffperoxyd an Harnstoff, Melamin oder Natriumborat, insbesondere Natriumpercarbonat oder Carbamid-Peroxyd. Wesentlich für die vorliegende Erfindung ist dabei, daß für die Mikroverkapselung ein Material verwendet wird, welches in saurer Umgebung stabil ist und das verkapselte Oxidationsmittel erst bei einem alkalischen pH-Wert freisetzt, da sonst die Urease vorzeitig desaktiviert würde. Erfindungsgemäß werden daher bevorzugt alkalilösliche Acrylharze für die Mikroverkapselung des Oxidationsmittels eingesetzt. Derartige Materialien sind beispielsweise unter der Bezeichnung Eudragit[R]S (Firma Röhm, Darmstadt), im Handel. Das erfindungsgemäße Haarfärbemittel enthält auf 100 Gewichtsteile der Grundmasse vorzugsweise 5 - 15 Gewichtsteile, insbesondere 8 - 11 Gewichtsteile des mikroverkapselten, Wasserstoffperoxid abgebenden Oxidationsmittels.

Die drei Komponenten werden getrennt hergestellt und bis zur Anwendung in getrennten Verpackungen oder vorzugsweise in einer geeigneten 3-Komponentenverpackung so gelagert, daß sie nicht miteinander in direkten Kontakt kommen können.

3

Die Grundmasse wird hergestellt, indem zunächst alle Bestandteile mit Ausnahme der Haarfarbzwischenprodukte homogen miteinander vermischt werden, vorzugsweise unter leichtem Erwärmen, insbesondere auf Temperaturen von 30 - 50° C. In speziellen Fällen kann es dabei zweckmäßig sein, zuerst eine Mischung aus Harnstoff und Puffergemisch herzustellen, die dann mit den übrigen Bestandteilen vereinigt wird. Vorzugsweise werden dann in das so erhaltene Gemisch die Haarfarbzwischenprodukte eingemischt.

Die Urease bzw. das durch trockenes Vermischen hergestellte Urease/Lipase-Gemisch liegt in Form eines feinverteilten Pulvers vor, das ggf. mit dem als Verdünnungsmittel verwendeten Feststoff vermischt wird.

Das Wasserstoffperoxid abgebende Oxydationsmittel wird nach einem üblichen Verfahren, vorzugsweise nach dem Wirbelschichtverfahren mikroverkapselt.

Das Färben von Haaren mit dem erfindungsgemäßen Haarfärbemittel erfolgt, indem die 3 Komponenten unmittelbar vor der Verwendung gemischt und das Gemisch anschließend auf das Haar aufgetragen und verteilt wird. Die erforderliche Menge an Haarfärbemittel ist, wie dem Fachmann bekannt ist, von der Länge des zu färbenden Haares abhängig. Die Anwendungstemperaturen liegen vorzugsweise in einem Bereich von 15 - 40° C. Nach einer Einwirkungszeit von vorzugsweise 15 - 45 Minuten, insbesondere 20 - 35 Minuten wird das Haarfärbemittel durch Ausspülen entfernt, ggf. mit einem milden Shampoo nachgewaschen und nochmals gespült, und das Haar getrocknet.

Das erfindungsgemäße Haarfärbemittel ist im Vergleich zu bisher bekannten Haarfärbemitteln milder und verträglicher, ohne daß Einbußen beim Färbeergebnis hingenommen werden müssen. Im Bereich der nachwachsenden Haaransätze zeigt sich im Gegenteil sogar eine verbesserte Färbung bei Anwendung des erfindungsgemäßen Mittels. Ein weiterer Vorteil ist die verminderte Geruchsbelästigung durch während der Anwendung auftretenden Ammoniakgeruch.

Die folgenden Beispiele sollen die Erfindung erläutern, ohne sie darauf zu beschränken. (GT bedeutet Gewichtsteile).

Beispiel 1

Tönungsgel:

Die Grundmasse wird durch Mischen der im folgenden angegebenen Komponenten unter leichtem Erwärmen (40°) hergestellt:

| Rohstoff | Gew.-% |
|---|---|
| Natriumsulfit | 0,60 |
| Isopropylalkohol | 10,00 |
| Cocosfettsäurepolydiethanolamid | 35,00 |
| Parfümölkomposition | 1,30 |
| Pufferlösung | 20,00 |
| Harnstoff | 5,00 |
| Cetyltrimethylammoniumchlorid (30%ig) | 10,00 |
| p-Phenylendiamindihydrochlorid | 0,07 |
| alpha-Naphtol | 0,04 |
| Chlorresorcin | 0,04 |
| Wasser | ad 100,00 |

Dabei werden die Haarfarbzwischenprodukte p-Phenylendiamindihydrochlorid, alpha-Naphtol und Chlorresorcin als letztes in die Lösung der übrigen Bestandteile eingearbeitet.

Die verwendete Pufferlösung ist eine wäßrige Lösung, die 0,95 Gew.-% Citronensäure und 4,30 Gew.-% Diammoniumhydrogenphosphat enthält.

Die Grundmasse weist einen pH-Wert von 6,5 auf.

Unmittelbar vor Gebrauch werden pro 100 GT Grundmasse 0,07 GT Urease und 8,6 GT mikroverkapseltes Carbamid-Peroxid Zugesetzt, die Komponenten vermischt und auf das Haar aufgetragen. Für eine Haarfärbung werden dabei ca. 50 g Grundmasse und die entsprechenden Mengen Urease und Carbamid-Peroxid benötigt. Es resultiert auf weißem Haar eine hellblonde Färbung.

Durch die Reaktion von Urease und Harnstoff steigt der pH-Wert von anfänglich 6,5 (Grundmasse) auf 8,9 bis 9,1.

Beispiel 2

Tönungsshampoo, hellblond:

Die Grundmasse hat folgende Zusammensetzung:

| Rohstoff: | Gew.-% |
|---|---|
| Natriumsulfit | 0,44 |
| Isopropylalkohol | 7,34 |
| Parfümölkomposition | 0,73 |
| EDTA | 0,15 |
| Kondensationsprodukt von Fettalkohol C12-C18 mit 3 Mol Ethylenoxid | 55,04 |
| Harnstoff | 5,62 |
| Pufferlösung (analog Beisp. 1) | 22,48 |
| p-Phenylendiamindihydrochlorid | 0,22 |
| Rersorcin | 0,009 |
| p-Nitro-o-aminophenol | 0,11 |
| o-Nitro-p-phenylendiamin | 0,01 |
| Wasser | ad 100,00 |

Die Herstellung erfolgt, indem zunächst die 5 zuerst genannten Bestandteile vermischt werden. Anschließend werden die mit dem Harnstoff gewünschte Pufferlösung und danach die Haarfarbzwischenprodukte zugegeben und alles homogen vermischt.

Unmittelbar vor Gebrauch werden pro 100 GT Grundmasse 0,1 GT Urease, 0,05 GT Lipase und 9,0 GT mikroverkapseltes Carbamid-Peroxid zugesetzt, die Komponenten vermischt und auf das Haar aufgetragen.

Beispiel 3


Haarfarbe:


Die Grundmasse hat folgende Zusammensetzung:

| Rohstoff | Gew.-% |
|---|---|
| Natriumsulfit | 0,50 |
| Cetyl-/Stearylalkohol | 10,50 |
| Natriumcetylstearylsulfat | 1,50 |
| Paraffinöl perliquidum . | 7,50 |
| Wollwachsalkohol | 3,75 |
| EDTA | 0,22 |
| Parfümölkomposition | 0,45 |
| Harnstoff | 5,00 |
| Pufferlösung (analog Beisp. 1) | 20,00 |
| p-Toluylendiaminsulfat | 1,00 |
| m-Phenylendiamin | 0,50 |
| Wasser | ad 100,00 |

Die Herstellung erfolgt, indem zunächst die 7 zuerst genannten Bestandteile mit dem Wasser vermischt werden. Anschließend werden die mit dem Harnstoff gemischte Pufferlösung und danach die Haarfarbzwischenprodukte zugegeben und alles homogen vermischt. Die Grundmasse weist einen pH-Wert von 6,3 auf.

Die Anwendung erfolgt wie in Beispiel 1 beschrieben, wobei pro 100 GT Grundmasse 0,1 GT Urease, 0,05 GT Lipase und 9,0 GT Carbamid-Peroxyd zugesetzt werden. Es resultiert auf weißem Menschenhaar eine blaue Färbung.

Beispiel 4

Tönungsshampoogel:

Die Grundmasse hat folgende Zusammensetzung:

| Rohstoff: | Gew.-% |
|---|---|
| Kondensationsprodukt aus Nonylphenol mit 4 Mol Ethylenoxid | 27,00 |
| Kondensationsprodukt aus Nonylphenol mit 9 Mol. Ethylenoxid | 23,00 |
| Isopropylalkohol | 16,44 |
| Ammoniumhydroxyd 25%ig | 11,00 |
| p-Toluylendiamin | 1,40 |
| m-Phenylendiamin | 0,05 |
| Resorcin | 0,06 |
| m-Aminophenol | 0,22 |
| p-Aminophenol | 0,23 |
| Ethylendiamintetraessigsäure | 0,2 |
| Harnstoff | 2,0 |
| Natriumsulfit | 1,0 |
| Wasser | 15,3 |
| Citronensäure | 0,38 |
| Diammoniumhydrogenphosphat | 1,72 |

Zur Herstellung der Grundmasse werden alle Rohstoffe der Reihe nach bei ca. 40°C homogen gemischt.

Diese Grundmasse wird unmittelbar in ihrer Verwendung mit 9 GT mikroverkapseltem Carbamid-Peroxid, 0,1 GT Urease und 0,05 GT Lipase vermischt und auf das Haar aufgetragen. Man erhält unter langsamen Ansteigen des pH-Wertes ein braunrotes Gel, das etwa 30 Min. färbewirksam ist. Es färbt zu 90% weiße Haare kastanienbraun.

**Patentansprüche**

1. Haarfärbemittel auf der Basis von Oxidationsfarben, gekennzeichnet durch

a) eine mittels eines geeigneten Puffergemisches im sauren Bereich gepufferte Grundmasse, die außerdem die Haarfarbzwischenprodukte und Harnstoff enthält,
b) Urease,
c) ein in einem gegen Säure beständigen in alkalischem Medium löslichen Material mikroverkapseltes, Wasserstoffperoxyd abgebendes Oxidationsmittel,
wobei die drei Komponenten (a), (b) und (c) erst unmittelbar vor dem Färbevorgang gemischt werden.

2. Haarfärbemittel gemäß Anspruch 1, dadurch gekennzeichnet, daß die Grundmasse auf einen pH-Wert von 4,9 - 6,9 gepuffert ist.

3. Haarfärbemittel gemäß Anspruch 1, dadurch gekennzeichnet, daß die Grundmasse 2 - 20 Gew.-% Harnstoff enthält.

4. Haarfärbemittel gemäß Anspruch 1, dadurch gekennzeichnet, daß es auf 100 Gewichtsteile Grundmasse 0,01 bis 0,20 Gewichtsteile, vorzugsweise 0,05 bis 0,15 Gewichtsteile Urease enthält.

5. Haarfärbemittel gemäß Anspruch 1, dadurch gekennzeichnet, daß es als Komponente (b) ein Gemisch aus Urease und Lipase enthält, wobei die Lipase in einer Menge von 0,01 bis 0,1 Gewichtsteilen, bezogen auf 100 Gewichtsteile Grundmasse, eingesetzt wird.

6. Haarfärbemittel gemäß Anspruch 1, dadurch gekennzeichnet, daß es auf 100 Gewichtsteile Grundmasse 5 - 15 Gewichtsteile insbesondere 8 - 11 Gewichtsteile des mikroverkapselten, Wasserstoffperoxyd abgebenden Oxydationsmittels enthält.

7. Haarfärbemittel gem. Anspruch 1, dadurch gekennzeichnet, daß das Wasserstoffperoxyd abgebende Oxydationsmittel Natriumpercarbonat oder Carbamid-Peroxyd ist.

8. Haarfärbemittel gemäß Anspruch 1, dadurch gekennzeichnet, daß als Puffergemisch eine wäßrige Lösung aus 0,7 bis 1,0 Gew.-% Citronensäure und 3,4 bis 4,3 Gew.-% Diammoniumhydrogenphosphat in einer Menge von 2 bis 25 Gew.-% der Grundmasse eingesetzt wird.

9. Verfahren zum Färben von Haaren auf der Basis von Oxydationsfarben, dadurch gekennzeichnet, daß man ein Mittel nach einem der Ansprüche 1 - 8 auf die Haare aufbringt, nach einer Einwirkungszeit von 15 - 45 Minuten spült, ggf. die Haare wäscht und nochmal spült und die Haare anschließend trocknet.

## Claims

1. Hair dyeing composition based on oxidation dyes, characterised by
   a) a foundation buffered in the acidic range by means of a suitable buffer mixture, which additionally contains the hair dyeing intermediates and urea,
   b) urease,
   c) an oxidation agent liberating hydrogen peroxide, microencapsulated in an acid-resistant material which is soluble in alkaline medium,
   where the three components (a), (b) and (c) are only mixed immediately before the dyeing process.

2. Hair dyeing composition according to Claim 1, characterised in that the foundation is buffered at a pH of 4.9-6.9.

3. Hair dyeing composition according to Claim 1, characterised in that the foundation contains 2 - 20% by weight of urea.

4. Hair dyeing composition according to Claim 1, characterised in that it contains 0.01 to 0.20 parts by weight, preferably 0.05 to 0.15 parts by weight, of urease to 100 parts by weight of foundation.

5. Hair dyeing composition according to Claim 1, characterised in that, as component (b) it contains a mixture of urease and lipase, where the lipase is employed in an amount of from 0.01 to 0.1 parts by weight, relative to 100 parts by weight of foundation.

6. Hair dyeing composition according to Claim 1, characterised in that it contains 5 - 15 parts by weight, in particular 8 - 11 parts by weight, of the microencapsulated oxidising agent liberating hydrogen peroxide to 100 parts by weight of foundation.

7. Hair dyeing composition according to Claim 1, characterised in that the oxidising agent liberating hydrogen peroxide is sodium percarbonate or carbamide peroxide.

8. Hair dyeing composition according to Claim 1, characterised in that the buffer mixture employed is an aqueous solution of 0.7 to 1.0% by weight of citric acid and 3.4 to 4.3% by weight of diammonium, hydrogen phosphate in an amount of from 2 to 25% by weight of the foundation.

9. Method for dyeing hair based on oxidation dyes, characterised in that a composition according to one of Claims 1 - 8 is applied to the hair and rinsed after a time of action of 15 - 45 minutes, the hair is optionally washed and rinsed again and the hair is then dried.

**Revendications**

1. Teinture pour les cheveux à base de colorants par oxydation, caractérisée par :
   a) une masse de base tamponnée à l'aide d'un mélange de tampons approprié dans la zone acide qui contient en outre les produits intermédiaires colorants capillaires et de l'urée,
   b) de l'uréase,
   c) un agent oxydant cédant du peroxyde d'hydrogène micro-encapsulé dans un matériau soluble en milieu alcalin, stable vis-à-vis des acides,
   dans lesquels les trois composants a), b) et c) sont mélangés en premier lieu, immédiatement avant le processus de couleur.

2. Teinture pour les cheveux selon la revendication 1, caractérisée en ce que la masse de base est tamponnée à une valeur de pH de 4,9 à 6,9.

3. Teinture pour les cheveux selon la revendication 1, caractérisée en ce que la masse de base contient de 2 à 20 % en poids d'urée.

4. Teinture pour les cheveux selon la revendication 1, caractérisée en ce qu'elle contient pour 100 parties en poids de masse de base, de 0,01 à 0,20 parties en poids - de préférence de 0,05 à 0,15 parties en poids - d'uréase.

5. Teinture pour les cheveux selon la revendication 1, caractérisée en ce qu'elle renferme en tant que composant b) un mélange d'uréase et de lipase dans lequel on met en jeu de la lipase en quantité de 0,01 à 0,1 partie en poids rapporté à 100 parties en poids de masse de base.

6. Teinture pour les cheveux selon la revendication 1, caractérisée en ce qu'elle contient pour 100 parties en poids de masse de base, 6 à 15 parties en poids, et en particulier 8 à 11 parties en poids d'agent d'oxydation cédant du peroxyde d'hydrogène, micro-encapsulé.

7. Teinture pour les cheveux selon la revendication 1, caractérisée en ce que l'agent d'oxydation cédant du peroxyde d'hydrogène est le percarbonate de sodium ou le peroxyde d'urée.

8. Teinture pour les cheveux caractérisée en ce que l'on met en oeuvre comme mélange tampon une solution aqueuse à base de 0,7 à 1 % en poids d'acide citrique et 3,4 à 4,3 % en poids d'hydrogénophosphate de diammonium en quantité allant de 2 à 25 % en poids de la masse de base.

9. Procédé pour teindre les cheveux à base de colorants par oxydation, selon l'une des revendications 1 à 8, caractérisé en ce que l'on applique un agent sur les cheveux, que l'on rince après un temps d'action de 15 à 45 mn, que l'on lave éventuellement les cheveux et rince à nouveau, et que l'on sèche ensuite les cheveux.